# EUROPEAN PATENT SPECIFICATION

(11) **EP 4 131 613 B1**
(45) Date of publication and mention of the grant of the patent: **21.01.2026**
(21) Application number: 21803818.0
(22) Date of filing: 27.04.2021
(51) Int. Cl.: H01M 50/30, H01M 50/342, H01M 50/317, H01M 50/116, H01M 10/48, G01N 33/00

(54) **SECONDARY BATTERY**
SEKUNDÄRBATTERIE
BATTERIE SECONDAIRE

(30) Priority: 13.05.2020 KR 20200057375
(43) Date of publication of application: 08.02.2023
(60) Divisional application: 25221561.1
(73) Proprietor: LG Energy Solution, Ltd., Seoul 07335 (KR)
(72) Inventor: KIM, Do Yul, Daejeon 34122 (KR); KO, Dong Wan, Daejeon 34122 (KR); LEE, Ki Young, Daejeon 34122 (KR)
(74) Representative: Plasseraud IP
(86) International application number: PCT/KR2021/005323
(87) International publication number: WO 2021/230536

(56) References cited:
- JP-A- 2007 134 535
- JP-A- 2010 153 356
- JP-A- 2012 015 121
- JP-A- 2020 056 445
- KR-A- 20150 061 996
- KR-A- 20180 113 855
- KR-A- 20180 113 855
- KR-A- 20200 025 909
- US-A1- 2005 277 017

## Description

### [Technical Field]

### CROSS-REFERENCE TO RELATED APPLICATION

The present invention claims the benefit of priority based on Korean Patent Application No. 10-2020-0057375 filed on May 13, 2020.

### TECHNICAL FIELD

The present invention relates to a secondary battery.

### [Background Art]

Secondary batteries are being researched and developed extensively recently, due to their being rechargeable unlike primary batteries and their potential for large capacity and small size. With increased technology development and demand for mobile devices, demand for secondary batteries as an electrical power source therefor is also rapidly increasing.

Secondary batteries are categorized into coin type batteries, cylindrical batteries, prismatic batteries and pouch type batteries depending on the shape of the battery case. A secondary battery accommodates an electrode assembly and an electrolyte solution. In a secondary battery, the electrode assembly mounted inside the battery case is a chargeable and dischargeable generating element comprised of a layered electrode and separator structure.

Electrode assemblies can be approximately categorized as jelly-rolls wherein a separator membrane is interposed between a sheet type cathode and anode on which an active material is applied, stacks wherein a plurality of cathodes and anodes are sequentially stacked with separator membranes interposed therebetween, and stacked/folded assemblies when stack type unit cells are wound using a long separating film.

A conventional pouch type battery is comprised of an electrode assembly accommodated within a pouch. When charging and discharging, such pouch type battery repeatedly swells and contracts due to gas generation. Here, if the generated gas remains within the pouch, it causes reduced battery performance and changes in volume, and this causes the problem of adverse impact on nearby batteries and structures. Further, if the gas generated exceeds the limit that can be accommodated by the pouch, a venting phenomenon wherein a structurally weak part ruptures and leaks gas occurs. Venting causes leakage of electrolyte solution, and the battery reaches the end of its life.

### [Document of Related Art]

Further prior art is described in KR 2014-0015647 A, JP 2020 056445 A, KR 2018 0113855 A, JP 2012 015121 A and US 2005/277017 A1.

### [Disclosure]

### [Technical Problem]

The object of the present invention is to provide a secondary battery equipped with a gas pocket to collect internal gas, and which is able to exhaust collected gas.

### [Technical Solution]

This object is accomplished with a secondary battery having the features of claim 1.

Dependent claims are directed on features of preferred embodiments of the invention.

### [Advantageous Effects]

According to the present invention, by providing double gas pockets for collecting internal gases, a large space for collecting internal gases can be secured, and direct contact between outside air and air inside a battery can be avoided.

Further, by forming gas exhaust ports which open at a certain pressure or higher on the respective dual gas pockets, it is possible to easily exhaust the gases accommodated in the gas pockets, preventing the rupture and leakage of gases through structurally weak parts of a battery case, and thereby preventing leakage of electrolyte solution and extending the life of the battery.

Further, by positioning a gas sensor within the gas pocket, it is possible to measure the occurrence of gases in a collection space, or to measure the constitution of the gas.

Further, by placing an air pressure sensor within the gas pocket, it is possible to measure air pressure in the collection space, which facilitates monitoring of battery status.

### [Description of Drawings]

FIG. 1 is a cross sectional diagram illustrating a secondary battery according to one embodiment of the present invention.
FIG. 2 is a cross section illustrating an essential part of the secondary battery according to one embodiment of the present invention.
FIG. 3 is a plan diagram illustrating the first and second exhaust ports of the secondary battery according to one embodiment of the present invention.
FIG. 4 is a cross section illustrating a closed status of the first and second exhaust ports of the secondary battery according to one embodiment of the present invention.
FIG. 5 is a cross section illustrating an open status of the first and second exhaust ports of the secondary battery according to one embodiment of the present invention.
FIG. 6 is a cross section illustrating a secondary battery according to another embodiment of the present invention.
FIG. 7 is a plan diagram illustrating the first and second exhaust ports of the secondary battery according to another embodiment of the present invention.
FIG. 8 is a cross section illustrating a closed status of the first and second exhaust ports of the secondary battery according to another embodiment of the present invention.
FIG. 9 is a cross section illustrating an open status of the first and second exhaust ports of the secondary battery according to another embodiment of the present invention.

### [Mode for Invention]

The purpose, specific benefits and novel characteristics of the present invention will become more evident from the following detailed description and preferred embodiments associated with the attached drawings. It shall be noted that in assigning reference numbers to the component elements of the respective drawings of the present specification, like elements are made to have like numbers anywhere possible even if they are represented in different drawings. Further, the present invention may be carried out in numerous different forms, and is not limited to the embodiments described herein. Also, in describing the present invention, detailed description of known art which may unnecessarily cloud the gist of the present invention shall be omitted.

### Secondary battery according to one embodiment

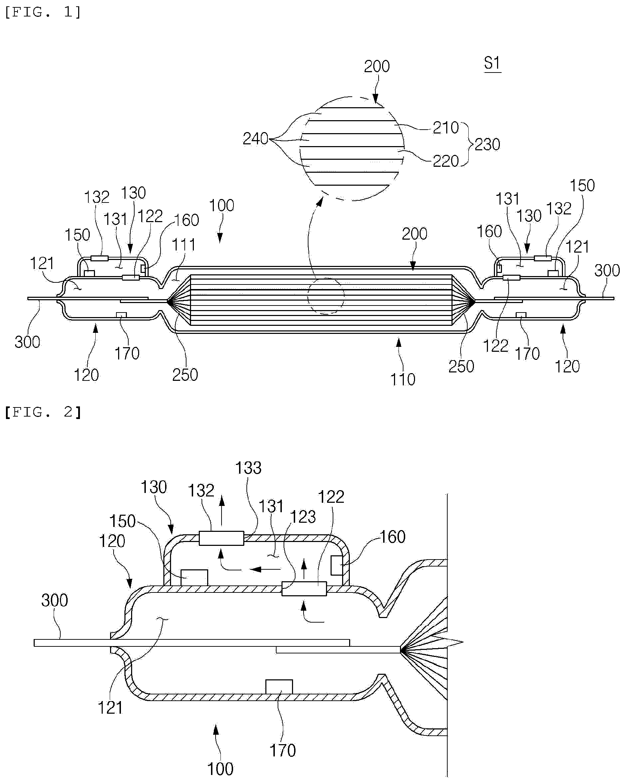

FIG. 1 is a cross sectional diagram illustrating the secondary battery according to one embodiment of the present invention, and FIG. 2 is a cross section illustrating an essential part of the secondary battery according to one embodiment of the present invention.

Referring to FIG. 1 and FIG. 2, the secondary battery S1 according to one embodiment of the present invention comprises an electrode assembly 200 and a battery case 100 in which the electrode assembly 200 is accommodated, where the battery case 100 comprises a first gas pocket 120 on which is formed a first collection space 121 and which is equipped with a first exhaust port 122 from which gas is exhausted, and a second gas pocket 130 on which is formed a second collection space 131 and which is equipped with a second exhaust port 132 from which gas is exhausted.

Further, the secondary battery S1 according to one embodiment of the present invention may further comprise a gas sensor 150 which detects occurrence of gases or measures the constitution of gas, and an air pressure sensor 160, 170 which measures air pressure.

In further detail, the electrode assembly 200, as a chargeable and dischargeable generating element, may consist of alternatingly stacked electrodes and separators. Here, an electrode tab 250 provided at an end of the electrode assembly 200 and an electrode lead may be connected to connect the electrode assembly 200 with an external device.

The electrode 230 may be comprised of a cathode 210 and an anode 220. Here, the electrode assembly 200 may consist of a structure wherein a cathode 210 / separator 240 / anode 220 are alternatingly stacked.

Also, the electrode lead 300 may comprise a cathode lead which is connected to a cathode tab provided at an end of the cathode 210, and an anode lead which is connected to an anode tab provided at an end of the anode 220.

The cathode 210 may comprise a cathode current collector, and a cathode active material laminating the cathode current collector.

The cathode current collector may be formed of an aluminum foil.

The cathode active material may be formed of lithium-manganese oxide, lithium-cobalt oxide, lithium-nickel oxide, lithium iron phosphate, or compounds and mixtures comprising at least one of these.

The anode 220 may comprise an anode current collector, and an anode active material laminating the anode current collector.

The anode current collector may be formed of, for example, a copper (Cu) foil.

The anode active material may be a compound or mixture comprising a graphite-based material.

The separator 240 is formed of insulating material and electrically insulates the cathode 210 and the anode 220. Here, the separator 240 may be formed of a microporous polyolefin resin membrane such as polyethylene or polypropylene.

The battery case 100 accommodates the electrode assembly 200.

The battery case 100 comprises a first gas pocket 120 on which a first collection space 121 is formed and which is equipped with a first exhaust port 122, and a second gas pocket 130 on which a second collection space 131 is formed and which is equipped with a second exhaust port 132.

Also, the battery case 100 further comprises a body 110 on which a housing 111 accommodating the electrode assembly 200 is formed.

The first gas pocket 120 has a first collection space 121 in which internal gases of the battery case 100 are collected, and a first exhaust port 122 from which gases in the first collection space 121 are discharged.

Further, the first gas pocket 120 extends along a side of the body 110 so that the housing 111 and the first collection space 121 are linked. Here, the first gas pocket 120 is positioned at an end of the body 110 where the electrode lead 300 is positioned. The first gas pocket 120 is positioned at both ends of the body 110.

The first exhaust port 122 is positioned above a first through-hole123 which penetrates the first collection space 121 and the second collection space 131, controlling the opening and closing of the first through-hole 123.

The second gas pocket 130 has a second collection space 131 in which gases exhausted from the first exhaust port 122 of the first gas pocket 120 are collected, and a second exhaust port 132 from which gases in the second collection space 131 are exhausted outward.

The second gas pocket 130 is provided above the first gas pocket 120 so that the second collection space 131 and the first collection space 121 are connected through the first through-hole 123.

The second exhaust port 132 is positioned above a second through-hole which penetrates the second collection space 131 and the exterior of the battery case 100, controlling the opening and controlling of the second through-hole 133.

Here, the first through-hole 123 and the second through-hole 133 may be provided at positions where they are not facing each other with respect to the direction in which gases are exhausted. Here, the first through-hole 123 and second through-hole 133 may be positioned at positions which do not face each other vertically.

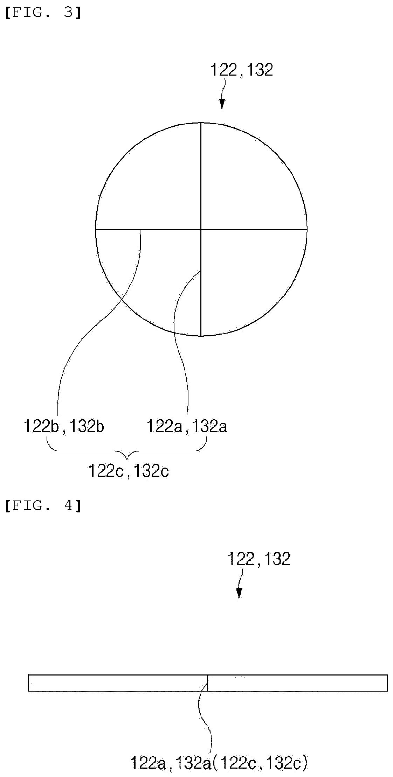

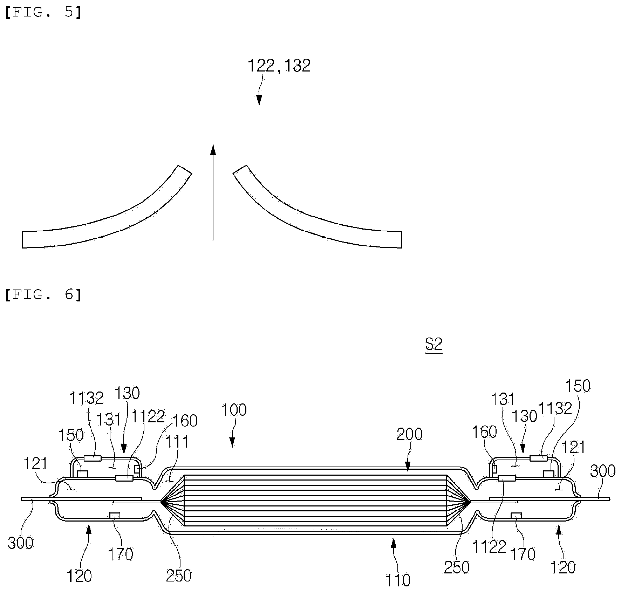

FIG. 3 is a plan diagram illustrating the first and second exhaust ports of the secondary battery according to one embodiment of the present invention, FIG. 4 is a cross section illustrating a closed status of the first and second exhaust ports of the secondary battery according to one embodiment of the present invention, and FIG. 5 is a cross section illustrating an open status of the first and second exhaust ports of the secondary battery according to one embodiment of the present invention.

Referring to FIG. 3 through FIG. 5, the first exhaust port 122 and the second exhaust port 132 are formed of elastic material and have a first opening 122c and a second opening 132c formed thereon, so that the first opening 122c and the second opening 132c open up at a certain pressure or higher, thereby opening the first through-hole 123 and the second through-hole 133.

Here, the elastic material may be formed of at least one of silicone, spandex, fluoroelastomer, ethylene, propylene rubber (EPR), styrene, butadiene rubber (SBR), and butyl rubber (PIB).

The first exhaust port 122 and second exhaust port 132 are provided to have different opening pressures. Here, the first exhaust port 122 and the second exhaust port 132 are provided to have different opening pressures by being formed of materials having different elasticity. Thereby, by preventing the simultaneous opening of the first exhaust port 122 and the second exhaust port 132, direct contact between air outside the battery with air inside the battery can be prevented.

Here, the first exhaust port 122 is provided with a greater opening pressure than that of the second exhaust port 132. Accordingly, the exhaust of gas through the first exhaust port 122 to the second exhaust port 132 is facilitated.

Referring to FIG. 3, the first opening 122c and the second opening 132c are formed of two to four incision lines 122a, 122b, 132a, 132b. Here, the first opening 122c and the second opening 132c are formed of cross ("+") shaped incision lines 122a, 122b, 132a, 132b.

Referring to FIG. 4, when the first collection space 121 and second collection space 131 are below a certain pressure, the first incision 122c and the second incision 132c close the first through-hole 123 and the second through-hole 133.

Referring to FIG. 5, when the first collection space 121 and the second collection space 131 are at a certain pressure or higher, the first incision 122c and the second incision 132c open up along the incision lines 122a, 122b, 132a, 132b to open the first through-hole 123 and the second through-hole 133.

Referring to FIG. 1 and FIG. 2, the gas sensor 150 is provided inside the second gas pocket 130 to detect the occurrence of gas in the second collection space 131 or measure the constitution of the gas. Here, the ability to detect a specific gas in the second gas pocket 130 through the gas sensor 150 enables alarms for specific purposes. Here, the gas sensor 150 may detect the occurrence of carbon dioxide (CO₂).

Further, the gas sensor 150 may, when gas is generated, trigger at least one of a notification signal, notification sound, or a notification light. Here, the gas sensor 150 may, for example, when carbon dioxide (CO₂) is detected, trigger at least one of a notification signal, notification sound, or a notification light. Here, the gas sensor 150 may comprise a notification speaker and notification LED to trigger a notification sound and notification light. Further, the gas sensor 150 is connected to a monitoring apparatus to forward a triggered notification signal.

The air pressure sensor 160, 170 may be provided inside at least one of the first gas pocket 120 or the second gas pocket 130 to measure air pressure. Here, the air pressure sensor 160, 170 may be positioned in the first gas pocket 120 and the second gas pocket 130 to measure the air pressure in the first collection space 121 and the second collection space 131.

Here, the air pressure sensor 160, 170 may, for example, when the measured air pressure value is equal to or higher than a certain pressure level, trigger at least one of a notification sound or a notification light.

Further, in another example, the air pressure sensor 160, 170 may forward a measured air pressure value to a monitoring apparatus. Here, the monitoring apparatus may be, for example a battery management system (BMS).

The secondary battery S1 according to one embodiment of the present invention, which is configured as described in the foregoing, by comprising a first gas pocket 120 and a second gas pocket 130 which collect and exhaust gases, is able to safely secure sufficient space for collection of internal gases, and also exhaust internal gases in a manner so that air outside the battery does not come into direct contact with air inside the battery.

Further, by forming, on the first gas pocket 120 and the second gas pocket 130 respectively, a first exhaust port 122 and a second exhaust port 132 which open and discharge gas at or above a certain pressure, it is possible to readily exhaust gases accommodated in the first gas pocket 120 and the second gas pocket 130, which in turn can extend the life of a battery.

Also, it is possible to position a gas sensor 150 in the second gas pocket 130 to detect the occurrence of gas in the second collection space 131 or measure the constitution of the gas.

### Secondary battery according to another embodiment

In the following, a secondary battery according to another embodiment of the present invention will be described.

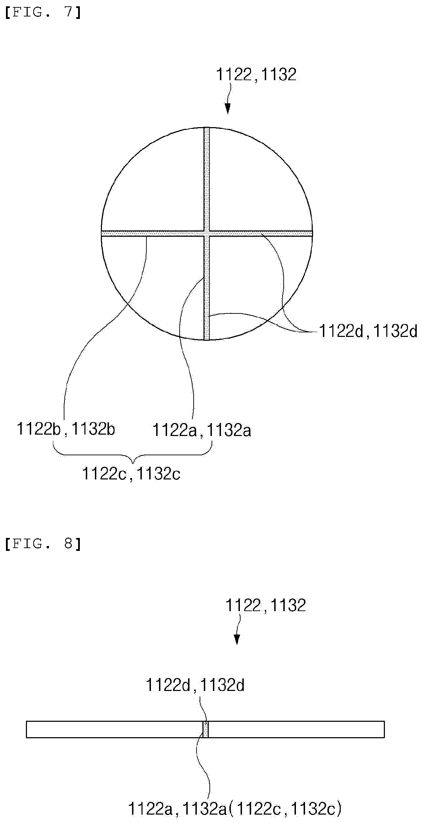

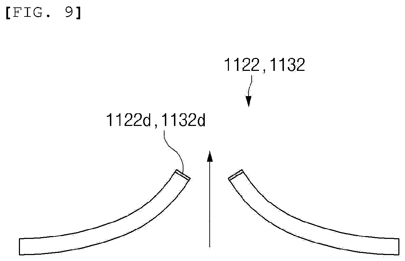

FIG. 6 is a cross section illustrating a secondary battery according to another embodiment of the present invention, FIG. 7 is a plan diagram illustrating the first and second exhaust ports of the secondary battery according to another embodiment of the present invention, FIG. 8 is a cross section illustrating a closed status of the first and second exhaust ports of the secondary battery according to another embodiment of the present invention, and FIG. 9 is a cross section illustrating an open status of the first and second exhaust ports of the secondary battery according to another embodiment of the present invention.

Referring to FIG. 6 through FIG. 9, the secondary battery S2 according to another embodiment of the present invention comprises an electrode assembly 200 and a battery case 100 which accommodates the electrode assembly 200, and the battery case 100 comprises a first gas pocket 120 on which a first collection space 121 is formed and which is equipped with a first exhaust port 1122 through which gas is exhausted, and a second gas pocket 130 on which a second collection space 131 is formed and which is equipped with a second exhaust port 132 through which gas is exhausted.

Further, the secondary battery S2 according to another embodiment of the present invention may further comprise a gas sensor 150 which detects the occurrence of gas or measures the constitution of gas, and an air pressure sensor 160, 170 which measures air pressure.

The secondary battery S2 according to another embodiment of the present invention, when compared against the secondary battery according to the first embodiment of the present invention described in the foregoing, differs in that an adhesive 1122d, 1132d is further disposed at the incision lines 1122a, 1122b, 1132a, 1132b of the first and second exhaust ports 1122, 1132. Therefore, in this second embodiment relating to this secondary battery S2, matters redundant with the previous first embodiment of a secondary battery will be omitted or stated briefly, with the description focusing on differences.

In further detail, the battery case 100 accommodates an electrode assembly 200.

The battery case 100 comprises a first gas pocket 120 on which a first collection space 121 is formed and which is equipped with a first exhaust port 1122 through which gas is exhausted, and a second gas pocket 130 on which a second collection space 131 is formed and which is equipped with a second exhaust port 132 through which gas is exhausted.

Further the battery case 100 further comprises a body 110 in which a housing 111 to accommodate the electrode assembly 200 is accommodated.

The first gas pocket 120 has formed thereon a first collection space 121 wherein internal gases of the battery case 100 are collected, and is equipped with a first exhaust port 1122 through which gases in the first collection space 121 are exhausted.

The first gas pocket 120 extends along a side of the body 110 so that the housing 111 and the first collection space 121 are connected.

On the second gas pocket 130, a second collection space 131 wherein gases exhausted from the first exhaust port 1122 of the first gas pocket 120 are collected is formed, and a second exhaust port 132 through which gases in the second collection space 131 are exhausted outward is provided.

The second gas pocket 130 is provided above the first gas pocket 120, so that the second collection space 131 and first collection space 121 are connected by a first through-hole.

The first exhaust port 1122 is positioned above a first through-hole which penetrates the first collection space 121 and the second collection space 131, controlling the opening and closing of the first through-hole.

The second exhaust port 132 is positioned above a second through-hole which penetrates the second collection space 131 and the outside of the battery case 100, controlling the opening and closing of the second through-hole.

The first exhaust port 1122 and the second exhaust port 132 have a first incision 1122c and a second incision 1132c formed thereon, and are formed of elastic material, so that at a certain pressure or higher, the first incision 1122c and the second incision 1132c open up, opening the first through-hole and the second through-hole.

The first incision 1122c and the second incision 1132c may be formed of two to four incision lines 1122a, 1122b, 1132a, 1132b. Here, the first incision 1122c and the second incision 1132c may be formed of cross ("+") shaped incision lines 1122a, 1122b, 1132a, 1132b.

The first exhaust port 1122 and the second exhaust port 132 are provided to have different opening pressures. Here, the first exhaust port 1122 and the second exhaust port 132 are formed of materials having different elasticity, so that the opening pressures of the first exhaust port 1122 and the second exhaust port 132 are different from each other. Here, the opening pressure of the first exhaust port 1122 is provided to be higher than the opening pressure of the second exhaust port 132.

The first exhaust port 1122 and the second exhaust port 132 further include an adhesive 1122d, 1132d disposed on the first incision 1122c and the second incision 1132c to additionally seal the first incision 1122c and the second incision 1132c. The adhesive strength sealing the first incision 1122c and the second incision 1132c is released when gas pressure causes the first incision 1122c and the second incision 1132c to open up.

Accordingly, through the adhesive 1122d, 1132d, the first incision 1122c and the second incision 1132c can be more tightly sealed, more effectively preventing the leakage of gas and electrolyte solution from the first incision 1122c of the first exhaust port 1122 and the second incision 1132c of the second exhaust port 132 at or below a certain pressure.

Further, a multitude of secondary batteries configured as described in the foregoing are electrically linked to form a battery pack.

Whereas the present invention has been described in detail with reference to specific embodiments, these embodiments are intended to exemplify the present invention, and the secondary battery according to the present invention is not limited thereto. The present invention may be carried out in various forms by a person having ordinary skill in the art.

Further, the specific scope of protection sought by the invention will become clear through the appended claims.

Further, the specific scope of protection sought by the invention shall become clear through the appended claims.

### <Explanation of Reference Numerals and Symbols>

S1, S2 : Secondary battery
100 : Battery case
110 : Body
111 : Housing
120 : First gas pocket
121 : First collection space
122, 1122 : First exhaust port
122a, 122b, 132a, 132b, 1122a, 1122b, 1132a, 1132b :
Incision line
122c, 1122c : First incision
123 : First through-hole
130 : Second gas pocket
131 : Second collection space
132, 1132 : Second exhaust port
132c, 1132c : Second incision
133 : Second through-hole
150 : Gas sensor
160, 170 : Air pressure sensor
200 : Electrode assembly
210 : Cathode
220 : Anode
230 : Electrode
240 : Separator
250 : Electrode tab
300 : Electrode lead
1122d, 1132d : Adhesive

## Claims

1. A secondary battery (S1) comprising an electrode assembly (200) in which electrodes and separators are alternatingly stacked;
and a battery case (100) in which the electrode assembly (200) is accommodated,
where the battery case (100) comprises a first gas pocket (120) on which a first collection space (121) for collecting gases in the battery case (100) is formed, and on which is provided a first exhaust port (122) from which gases in the first collection space (121) are exhausted; and
a second gas pocket (130) on which a second collection space (131) for collecting gases exhausted from the first exhaust port (122) is formed, and on which is provided a second exhaust port (132) for outward exhaust of gas in the second collection space (131),
**characterized by** an air pressure sensor (170) provided inside the first gas pocket (120) to measure air pressure.

2. The secondary battery (S1) of Claim 1,
wherein the first exhaust port (122) is positioned above a first through-hole (123) penetrating the first collection space (121) and the second collection space (131) to control the opening and closing of the first through-hole (123),
and the second exhaust port (132) is positioned above a second through-hole (133) penetrating the second collection space (131) and the outside of the battery case (100) to control the opening and closing of the second through-hole (133).

3. The secondary battery (S1) of Claim 2, wherein
the battery case (100) comprises a body (110) in which a housing (111) accommodating the electrode assembly (200) is formed,
the first gas pocket (120) extends along a side of the body (110) so that the housing (111) and the first collection space (121) are connected,
and the second gas pocket (130) is provided above the first gas pocket (120) so that the second collection space (131) and the first collection space (121) are connected by the first through-hole (123).

4. The secondary battery (S1) of Claim 2,
wherein the first exhaust port (122) and the second exhaust port (132) have formed thereon a first incision (122c) and a second incision (132c) and are themselves formed of elastic material, so that at or above a certain pressure, the first incision (122c) and the second incision (132c) open up and open the first through-hole (123) and the second through-hole (133).

5. The secondary battery (S1) of Claim 4,
wherein the first incision (122c) and the second incision (132c) are comprised of 2 to 4 incision lines (122a, 132a, 122b, 132b).

6. The secondary battery (S1) of Claim 4,
wherein the first incision (122c) and the second incision (132c) are comprised of cross ("+") shaped incision lines (122a, 132a, 122b, 132b).

7. The secondary battery (S1) of Claim 4, wherein the first exhaust port (122) and the second exhaust port (132) further comprise an adhesive (1122d, 1132d) disposed on the first incision (122c) and the second incision (132c) to additionally seal the first incision (122c) and the second incision (132c), and where the adhesive force sealing the first incision (122c) and the second incision (132c) is released when the first incision (122c) and the second incision (132c) are opened up by gas pressure.

8. The secondary battery (S1) of Claim 2,
wherein the first exhaust port (122) and the second exhaust port (132) are provided to have different opening pressures from each other.

9. The secondary battery (S1) of Claim 2,
wherein the first exhaust port (122) and the second exhaust port (132) are formed of materials having different elasticities from each other, so that the first exhaust port (122) and the second exhaust port (132) are provided to have different opening pressures from each other.

10. The secondary battery (S1) of Claim 8, wherein the opening pressure of the first exhaust port (122) is provided to be greater than the opening pressure of the second exhaust port (132).

11. The secondary battery of Claim 1,
further comprising a gas sensor (150) provided inside the second gas pocket (130) to detect occurrence of gas within the second collection space (131) or to measure the constitution of gas.

12. The secondary battery (S1) of Claim 11,
wherein the gas sensor (150) detects the occurrence of carbon dioxide (CO₂).

13. The secondary battery (S1) of Claim 11,
wherein the gas sensor (150) triggers at least one of a notification signal, notification sound or notification light when gas occurs.

14. The secondary battery (S1) of claim 1,
further comprising an air pressure sensor (160) provided inside the second gas pocket (130) to measure air pressure.

15. A battery pack comprising the secondary battery (S1) stated in any one of Claim 1 through Claim 14.

## Patentansprüche

1. Sekundärbatterie (S1), umfassend eine Elektrodenanordnung (200), in welcher Elektroden und Separatoren alternierend gestapelt sind;
und ein Batteriegehäuse (100), in welchem die Elektrodenanordnung (200) aufgenommen ist,
wobei das Batteriegehäuse (100) eine erste Gastasche (120) umfasst, an welcher ein erster Sammelraum (121) zum Sammeln von Gasen in dem Batteriegehäuse (100) gebildet ist und an welcher eine erste Auslassöffnung (122) bereitgestellt ist, von welcher Gase in dem ersten Sammelraum (121) ausgelassen werden; und
eine zweite Gastasche (130) umfasst, an welcher ein zweiter Sammelraum (131) zum Sammeln von Gasen, welche von der ersten Auslassöffnung (122) ausgelassen werden, gebildet ist und an welcher eine zweite Auslassöffnung (132) für einen Auslass nach außen von Gas in dem zweiten Sammelraum (131) bereitgestellt ist,
**gekennzeichnet durch** einen Luftdrucksensor (170), welcher in der ersten Gastasche (120) bereitgestellt ist, um einen Luftdruck zu messen.

2. Sekundärbatterie (S1) nach Anspruch 1,
wobei die erste Auslassöffnung (122) über einem ersten Durchgangsloch (123) positioniert ist, welches den ersten Sammelraum (121) und den zweiten Sammelraum (131) durchdringt, um das Öffnen und Schließen des ersten Durchgangslochs (123) zu steuern, und
die zweite Auslassöffnung (132) über einem zweiten Durchgangsloch (133) positioniert ist, welches den zweiten Sammelraum (131) und das Äußere des Batteriegehäuses (100) durchdringt, um das Öffnen und Schließen des zweiten Durchgangslochs (133) zu steuern.

3. Sekundärbatterie (S1) nach Anspruch 2, wobei
das Batteriegehäuse (100) einen Körper (110) umfasst, in welchem ein Gehäuse (111), welches die Elektrodenanordnung (200) aufnimmt, gebildet ist,
sich die erste Gastasche (120) entlang einer Seite des Körpers (110) erstreckt, so dass das Gehäuse (111) und der erste Sammelraum (121) verbunden sind, und
die zweite Gastasche (130) über der ersten Gastasche (120) bereitgestellt ist, so dass der zweite Sammelraum (131) und der erste Sammelraum (121) durch das erste Durchgangsloch (123) verbunden sind.

4. Sekundärbatterie (S1) nach Anspruch 2,
wobei die erste Auslassöffnung (122) und die zweite Auslassöffnung (132) darauf einen ersten Einschnitt (122c) und einen zweiten Einschnitt (132c) gebildet haben und selbst aus einem elastischen Material gebildet sind, so dass an oder über einem bestimmten Druck, sich der erste Einschnitt (122c) und der zweite Einschnitt (132c) öffnen und das erste Durchgangsloch (123) und das zweite Durchgangsloch (133) öffnen.

5. Sekundärbatterie (S1) nach Anspruch 4,
wobei der erste Einschnitt (122c) und der zweite Einschnitt (132c) aus 2 bis 4 Einschnittlinien (122a, 132a, 122b, 132b) umfasst sind.

6. Sekundärbatterie (S1) nach Anspruch 4,
wobei der erste Einschnitt (122c) und der zweite Einschnitt (132c) aus kreuz-("+")-förmigen Einschnittlinien (122a, 132a, 122b, 132b) umfasst sind.

7. Sekundärbatterie (S1) nach Anspruch 4, wobei die erste Auslassöffnung (122) und die zweite Auslassöffnung (132) ferner ein Haftmittel (1122d, 1132d) umfassen, welches an dem ersten Einschnitt (122c) und dem zweiten Einschnitt (132c) angeordnet ist, um den ersten Einschnitt (122c) und den zweiten Einschnitt (132c) zusätzlich abzudichten, und wobei die Haftmittelkraft, welche den ersten Einschnitt (122c) und den zweiten Einschnitt (132c) abdichtet, gelöst wird, wenn der erste Einschnitt (122c) und der zweite Einschnitt (132c) durch einen Gasdruck geöffnet werden.

8. Sekundärbatterie (S1) nach Anspruch 2,
wobei die erste Auslassöffnung (122) und die zweite Auslassöffnung (132) bereitgestellt sind, um unterschiedliche Öffnungsdrücke voneinander aufzuweisen.

9. Sekundärbatterie (S1) nach Anspruch 2,
wobei die erste Auslassöffnung (122) und die zweite Auslassöffnung (132) aus Materialien gebildet sind, welche unterschiedliche Elastizitäten voneinander aufweisen, so dass die erste Auslassöffnung (122) und die zweite Auslassöffnung (132) bereitgestellt sind, um unterschiedliche Öffnungsdrücke voneinander aufzuweisen.

10. Sekundärbatterie (S1) nach Anspruch 8, wobei der Öffnungsdruck der ersten Auslassöffnung (122) bereitgestellt ist, um größer zu sein als der Öffnungsdruck der zweiten Auslassöffnung (132).

11. Sekundärbatterie (S1) nach Anspruch 1,
ferner umfassend einen Gassensor (150), welcher in der zweiten Gastasche (130) bereitgestellt ist, um ein Auftreten von Gas innerhalb des zweiten Sammelraums (131) zu detektieren oder um die Zusammensetzung von Gas zu messen.

12. Sekundärbatterie (S1) nach Anspruch 11,
wobei der Gassensor (150) das Auftreten von Kohlenstoffdioxid (CO₂) detektiert.

13. Sekundärbatterie (S1) nach Anspruch 11,
wobei der Gassensor (150) wenigstens eines aus einem Benachrichtigungssignal, einem Benachrichtigungston oder einem Benachrichtigungslicht auslöst, wenn Gas auftritt.

14. Sekundärbatterie (S1) nach Anspruch 1,
ferner umfassend einen Luftdrucksensor (160), welcher in der zweiten Gastasche (130) bereitgestellt ist, um einen Luftdruck zu messen.

15. Batteriepack, umfassend die Sekundärbatterie (S1), wie in einem aus Anspruch 1 bis Anspruch 14 ausgeführt.

## Revendications

1. Batterie secondaire (S1) comprenant un ensemble d'électrodes (200) dans lequel des électrodes et des séparateurs sont empilés alternativement ;
et un boîtier de batterie (100) dans lequel l'ensemble d'électrodes (200) est logé,
où le boîtier de batterie (100) comprend une première poche de gaz (120) sur laquelle est formé un premier espace de collecte (121) pour collecter des gaz dans le boîtier de batterie (100), et sur laquelle est prévu un premier orifice d'échappement (122) par lequel sont évacués les gaz dans le premier espace de collecte (121) ; et
une deuxième poche de gaz (130) sur laquelle est formé un deuxième espace de collecte (131) pour collecter des gaz évacués du premier orifice d'échappement (122), et sur laquelle est prévu un deuxième orifice d'échappement (132) pour évacuer vers l'extérieur les gaz dans le deuxième espace de collecte (131),
**caractérisée par** un capteur de pression d'air (170) disposé à l'intérieur de la première poche de gaz (120) pour mesurer une pression d'air.

2. Batterie secondaire (S1) selon la revendication 1,
dans laquelle le premier orifice d'échappement (122) est positionné au-dessus d'un premier trou traversant (123) pénétrant dans le premier espace de collecte (121) et le deuxième espace de collecte (131) pour commander l'ouverture et la fermeture du premier trou traversant (123),
et le deuxième orifice d'échappement (132) est positionné au-dessus d'un deuxième trou traversant (133) pénétrant dans le deuxième espace de collecte (131) et l'extérieur du boîtier de batterie (100) pour commander l'ouverture et la fermeture du deuxième trou traversant (133).

3. Batterie secondaire (S1) selon la revendication 2, dans laquelle le boîtier de batterie (100) comprend un corps (110) dans lequel est formé un logement (111) accueillant l'ensemble d'électrodes (200),
la première poche de gaz (120) s'étend le long d'un côté du corps (110) de sorte que le logement (111) et le premier espace de collecte (121) soient reliés,
et la deuxième poche de gaz (130) est prévue au-dessus de la première poche de gaz (120) de sorte que le deuxième espace de collecte (131) et le premier espace de collecte (121) soient reliés par le premier trou traversant (123).

4. Batterie secondaire (S1) selon la revendication 2,
dans laquelle le premier orifice d'échappement (122) et le deuxième orifice d'échappement (132) présentent une première incision (122c) et une deuxième incision (132c) formées sur ceux-ci et sont eux-mêmes formés d'un matériau élastique, de sorte qu'à une certaine pression ou au-delà, la première incision (122c) et la deuxième incision (132c) s'ouvrent et permettent l'ouverture du premier trou traversant (123) et du deuxième trou traversant (133).

5. Batterie secondaire (S1) selon la revendication 4,
dans laquelle la première incision (122c) et la deuxième incision (132c) sont constituées de 2 à 4 lignes d'incision (122a, 132a, 122b, 132b).

6. Batterie secondaire (S1) selon la revendication 4,
dans laquelle la première incision (122c) et la deuxième incision (132c) sont constituées de lignes d'incision (122a, 132a, 122b, 132b) en forme de croix (« + »).

7. Batterie secondaire (S1) selon la revendication 4, dans laquelle le premier orifice d'échappement (122) et le deuxième orifice d'échappement (132) comprennent en outre un adhésif (1 122d, 1132d) disposé sur la première incision (122c) et la deuxième incision (132c) pour sceller davantage la première incision (122c) et la deuxième incision (132c), et où la force d'adhérence scellant la première incision (122c) et la deuxième incision (132c) est libérée lorsque la première incision (122c) et la deuxième incision (132c) sont ouvertes par une pression de gaz.

8. Batterie secondaire (S1) selon la revendication 2,
dans laquelle le premier orifice d'échappement (122) et le deuxième orifice d'échappement (132) sont prévus pour présenter des pressions d'ouverture différentes.

9. Batterie secondaire (S1) selon la revendication 2,
dans laquelle le premier orifice d'échappement (122) et le deuxième orifice d'échappement (132) sont formés de matériaux présentant des élasticités différentes, de sorte que le premier orifice d'échappement (122) et le deuxième orifice d'échappement (132) soient conçus pour présenter des pressions d'ouverture différentes.

10. Batterie secondaire (S1) selon la revendication 8, dans laquelle la pression d'ouverture du premier orifice d'échappement (122) est supérieure à la pression d'ouverture du deuxième orifice d'échappement (132).

11. Batterie secondaire selon la revendication 1,
comprenant en outre un capteur de gaz (150) disposé à l'intérieur de la deuxième poche de gaz (130) pour détecter la présence de gaz dans le deuxième espace de collecte (131) ou pour mesurer la composition d'un gaz.

12. Batterie secondaire (S1) selon la revendication 11,
dans laquelle le capteur de gaz (150) détecte la présence de dioxyde de carbone (CO₂).

13. Batterie secondaire (S1) selon la revendication 11,
dans laquelle le capteur de gaz (150) déclenche au moins un parmi un signal de notification, un son de notification ou un voyant de notification lorsque du gaz est détecté.

14. Batterie secondaire (S1) selon la revendication 1,
comprenant en outre un capteur de pression d'air (160) disposé à l'intérieur de la deuxième poche de gaz (130) pour mesurer la pression d'air.

15. Bloc-batterie comprenant la batterie secondaire (S1) selon l'une quelconque des revendications 1 à 14.
